# EUROPÄISCHE PATENTSCHRIFT

(11) **EP 0 640 326 B1**
(45) Veröffentlichungstag und Bekanntmachung des Hinweises auf die Patenterteilung: **12.01.2000**
(21) Anmeldenummer: 93810617.6
(22) Anmeldetag: 30.08.1993
(51) Int. Cl.: A61F 2/36, A61F 2/34, A61L 27/00

(54) **Element zur vorübergehenden Erhöhung der Steifigkeit einer orthopädischen Prothese**
Element for temporarily increasing the rigidity of a prosthesis
Elément pour augmenter temporairement la rigidité d'une prothèse

(43) Veröffentlichungstag der Anmeldung: 01.03.1995
(73) Patentinhaber: Sulzer Orthopädie AG, 6340 Baar (CH)
(72) Erfinder: Maumy, JEAN Dr., F-82 000 Montauban (FR); Baege, Roland, CH-8542 Wiesendangen (CH)
(74) Vertreter: Heinen, Detlef

(56) Entgegenhaltungen:
- EP-A- 0 176 711
- EP-A- 0 321 389
- EP-A- 0 346 270
- EP-A- 0 404 716
- EP-A- 0 445 068
- EP-A- 0 495 341
- WO-A-85/05027
- WO-A-93/08770
- US-A- 4 329 743
- US-A- 5 163 960
- US-A- 5 207 712

## Beschreibung

Die Erfindung bezieht sich auf eine orthopädische Prothese gemäss dem Oberbegriff von Anspruch 1. Eine gattungsgemäße Prothese ist aus der EP-A-0 404 716 bekannt.

Aus der EP 0 445 068 A1 ist eine orthopädische Prothese, insbesondere eine künstliche Hüftgelenkpfanne bekannt, die aus einer Innenschale sowie aus einer Aussenschale besteht, wobei die Wandstärke der Aussenschale wesentlich dünner ist als die Wandstärke der Innenschale. Dabei ist die metallische Aussenschale so dünn gehalten, dass sie sich einerseits nach dem Einwachsen im Knochengewebe kleinen Veränderungen des Knochengewebes elastisch anpassen kann. Andererseits werden Kräfte von der relativ starren metallischen Innenschale auf die Aussenschale übertragen, sodass die Aussenschale sich entsprechend den Reaktionskräften im Knochengewebe und entsprechend ihrer Steifigkeit deformiert.
Die elastischen Eigenschaften einer orthopädischen Prothese können sich unmittelbar nach der Implantation während einer begrenzten Zeit als nachteilig erweisen. Beispielsweise können die elastischen Eigenschaften das Anwachsen des Knochens an die Prothese behindern.
So weist auch die bekannte künstliche Hüftgelenkpfanne den Nachteil auf, dass sich die Aussenschale unter ungünstigen Bedingungen, insbesondere beim Einwirken grösserer Kräfte, deformieren kann, was das Anwachsen des Knochengewebes in die Aussenfläche der Aussenschale verzögern oder behindern kann.

Der Erfindung liegt die Aufgabe zugrunde, eine Prothese zu schaffen, die eine zeitlich begrenzte, erhöhte Steifigkeit aufweist.

Erfindungsgemäss wird diese Aufgabe gelöst gemäss den Merkmalen von Anspruch 1. Die Unteransprüche beziehen sich auf weitere, vorteilhafte Ausführungsformen der erfindungsgemässen Prothese.

Das Element in der Prothese besteht aus einem biologisch abbaubaren Körper, der vor oder während der Implantation der orthopädischen Prothese derart in eine Ausnehmung der Prothese eingefügt wird, dass sich die Steifigkeit der Prothese erhöht. So lässt sich zum Beispiel bei der aus der EP 0 445 068 bekannten, künstlichen Hüftgelenkpfanne ein abbaubares Element in den Raum zwischen Innen- und Aussenschale einfügen, sodass das Element eine Stützfunktion ausübt. Das Element bewirkt eine mechanische Verbindung zwischen Innen- und Aussenschale und lässt somit eine zusätzliche mechanische Koppelung zwischen Innen- und Aussenschale entstehen, was die elastischen Eigenschaften respektive die Bewegungsmöglichkeiten der Aussenschale reduziert. Die Fähigkeit des biologisch abbaubaren Elementes, eine Kraft zu übertragen, reduziert sich nach erfolgter Implantation zunehmend, sodass die Aussenschale der künstlichen Hüftgelenkpfanne, entsprechend den konstruktiv bedingten, mechanischen Eigenschaften, zunehmend elastischere Eigenschaften aufweist. Nach einer gewissen Zeitspanne nach der Implantation ist das biologisch abbaubare Element soweit abgebaut, dass es keine Kraft mehr überträgt.
Der Vorteil des erfindungsgemässen Elementes ist primär darin zu sehen, dass es der orthopädischen Prothese während einer unmittelbar der Implantation folgenden Zeit eine erhöhte Steifigkeit verleiht, und somit zum Beispiel Relativbewegungen an der Oberfläche zwischen Knochen und Prothese vermindert. Längerfristig nach der Implantation baut sich das Element biologisch ab, sodass sich die Steifigkeit der prothetischen Vorrichtung zunehmend reduziert, und das nach erfolgtem Einwachsen erwünschte elastische Verhalten eintritt. Ein weiterer Vorteil ist darin zu sehen, dass kein zweiter chirurgischer Eingriff notwendig ist, um das Element zu entfernen.

Im folgenden wird die Erfindung anhand von Ausführungsbeispielen beschrieben. Es zeigen:
- Fig. 1a: eine Seitenansicht eines Schaftes einer Gelenkendoprothese;
- Fig. 1b: ein Schnitt durch die Prothese entlang der Linie A-A;
- Fig. 1c: ein Element zur Erhöhung der Steifigkeit;
- Fig. 1d: ein Jochteil einer weiteren Gelenkendoprothese;
- Fig. 1e: eine weitere Ausführungsform eines Elementes zur Erhöhung der Steifigkeit;
- Fig. 2a: eine Aussenschale einer künstlichen Hüftgelenkpfanne;
- Fig. 2b: ein Element zur Erhöhung der Steifigkeit;
- Fig. 2c: eine Untenansicht einer Aussenschale einer künstlichen Hüftgelenkpfanne;
- Fig. 3a: eine Draufsicht auf eine weitere Aussenschale einer künstlichen Hüftgelenkpfanne;
- Fig. 3b: eine Seitenansicht eines Schnittes (I-I) durch die Hüftgelenkpfanne.

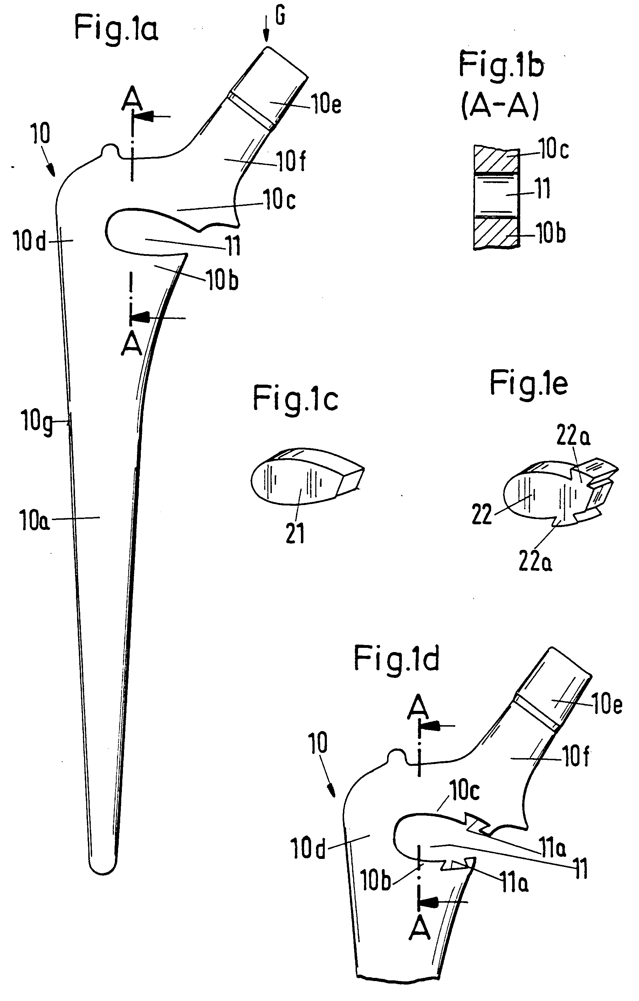

Fig. 1a zeigt eine Seitenansicht einer Gelenkendoprothese 10. Der gerade Schaft 10a erweitert sich konisch von seinem distalen Ende nach proximal. Die laterale Schmalseite 10g führt in einem Bogen zu einer horizontalen Schulter am proximalen Schaftende, die ihrerseits in einen Prothesenhals 10f übergeht. Dieser trägt einen konischen Zapfen 10e für die Aufnahme eines nicht dargestellten Gelenkkopfes. Der Schaft 10a bildet im Bereich des Bogens und der horizontalen Schulter ein Jochteil, das eine Ausnehmung 11 aufweist. Die Ausnehmung 11 ist von zwei anliegende Bereiche 10b, 10c sowie einem elastisch federnden Bereich 10d begrenzt. Beim Auftreten einer stossartigen Gewichtskraft G bewirkt dies eine leichte Biegung im Jochteil und somit eine Dämpfung der einwirkenden Kraft in distaler Richtung, sodass stossartige Belastungen ohne lokale Spannungsspitzen auf das Knochengewebe übertragbar sind. Gleichzeitig erhöhen sich jedoch die in einer transversalen Ebene auftretenden Biegekräfte, die von der Prothese auf das Knochengewebe übertragen werden. Die durch die federnden Eigenschaften des Jochteils erzeugten Kräft können das Einwachsen des Knochengewebes an die Prothese erschweren.
Ein vertikaler Schnitt entlang der Linie A-A ist in Fig. 1b dargestellt, woraus die Ausnehmung 11 im Jochteil sowie die beiden anliegenden Bereiche 10b, 10c ersichtlich sind. Das federnde, elastische Verhalten im Jochteil zwischen Prothesenschaft 10a und Prothesenhals 10f kann entsprechend der Ausgestaltung der Ausnehmung 11 in einem weiten Bereich variiert werden. Ein in Fig. 1c dargestelltes biologisch abbaubares Element 21 ist der Ausnehmung 11 entsprechend angepasst ausgestaltet, sodass das Element 21 vor oder während der Implantation der Prothese 10 form- oder kraftschlüssig in die Ausnehmung 11 einlegbar ist. Nicht dargestellt aber selbstverständlich ist eine gegenseitge Ausgestaltung der anliegenden Bereiche 10b, 10c sowie des Elementes 21 derart, dass das Element 21 in der Ausnehmung 11 fixierbar ist, zum Beispiel durch eingebrachte Nuten und Vorsprünge, sodass das Element 21 in die Ausnehmung 11 einschnappt.
Die Lastaufnahmefähigkeit des Elementes 21 ist abhängig von den verwendeten Materialien und bewirkt eine Reduzierung der Federwirkung im Jochbereich, beziehungsweise im Übergangsbereich zwischen Prothesenschaft 10a und Prothesenhals 10f, sodass sich die Steifigkeit der gesamten Prothese 10 erhöht. Das in Fig. 1c dargestellte Element 21 lässt sich nur auf Druck belasten. In Fig. 1e ist ein weiteres Element 22 dargestellt, das über Eingriffselemente 22a verfügt, die derart in die Ausnehmungen 11a der in Fig. 1d dargestellten Prothese 10 zu liegen kommen, dass das Element 22 sowohl auf Zug wie auf Druck belastbar ist.

Der Körper 21, 22 besteht aus einem biokompatiblen, biologisch resorbierbaren Polymer. Solche resorbierbaren Polymere und Copolymere sind aus der Literatur bekannt und beinhalten z.B. Polymilchsäure, Polyglykolsäure, Polydioxanon, Polycaprolacton, Knochenwachstumsfaktoren.
Die Körper 21, 22 können wahlweise mit osteogenetischen anorganischen Zusatzstoffen wie Hydroxylapatit oder Tricalciumphospat zum kontrollierten Einwachsen in die durch das abgebaute Polymer erzeugten Poren versehen sein. Weiter können den Polymeren Antibiotika oder knochenstimulierende pharmazeutische Wirkstoffe, wie Etidronat, zur Vermeidung von Osteoporose beigemischt werden.

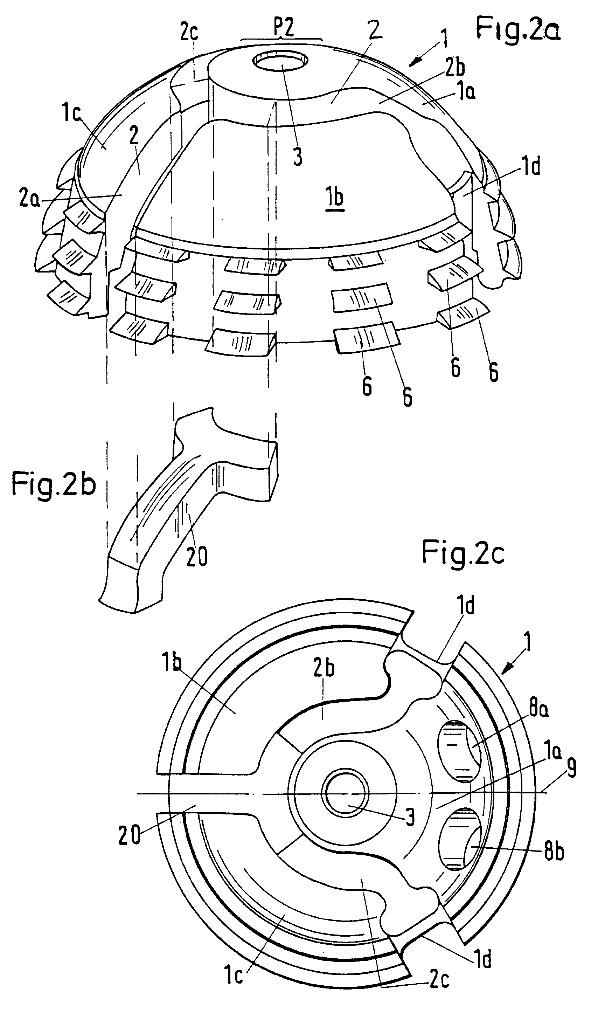

In Fig. 2b ist ein weiteres biokompatibles, biologisch resorbierbares beziehungsweise biologisch abbaubares Element 20 dargestellt, das dazu dient, die elastischen Eigenschaften einer in Fig. 2a und 2c dargestellten Aussenschale 1 einer Hüftgelenkpfanne während einer der Implantation folgenden Zeitperiode zu reduzieren. Die Figuren 2a und 2c zeigen eine metallische Aussenschale 1, die im wesentlichen aus einem Verankerungsbereich 1a sowie zwei über federnde Verbindungselemente 1d mit dem Verankerungsbereich 1a verbundene Aussenschalensegmente 1b und 1c gebildet wird. Der Verankerungsbereich 1a lässt sich mit Verankerungshilfen wie Dornen 6 oder mit Knochenschrauben, die durch die Bohrungen 8a, 8b verlaufen, im Knochengewebe primär verankern. Im Bereich des Poles P2 ist eine Zentrier- oder Führungsbohrung 3 angebracht, an der die Innenschale befestigbar ist.
Die Aussenschale 1 weist somit Ausnehmungen 2 auf, die bewirken, dass die Aussenschale 1 insbesondere in Umfangsrichtung elastisch zusammenpressbar oder spreizbar ist und somit federne, elastische Eigenschaften aufweist, die sich während der Phase des Einwachsens des Knochengewebes an die Aussenschale 1 nachteilig auswirken können. Um die Elastizität der Aussenschale 1 zu verringern sind Elemente 20 form- oder kraftschlüssig in den Spalt 2 einführbar. Im vorliegenden Ausführungsbeispiel weist die Aussenschale 1 zwei S-förmig verlaufende Spalten 2b, 2c zwischen dem Verankerungsbereich 1a und den Aussenschalensegmenten 1b, 1c auf, sowie ein in Richtung eines Meridiankreises verlaufender radialer Spalt 2 zwischen den Aussenschalensegmenten 1b und 1c. Wie in Fig. 2a dargestellt, lässt sich das Element 20 in den Spalt 2 hineinschieben, wobei das Element 20 und die Aussenschale 1 auch mit Mitteln versehbar sind, um die gegenseitige Lage zu definieren, zum Beispiel derart, dass das Element 20 in die Aussenschale 1 einschnappt. Nach der Implantation wird das Element 20 biologisch abgebaut, sodass das elastische Verhalten der Aussenschale 1 längere Zeit nach der Implantation durch das Element 20 nicht mehr beeinflusst wird.

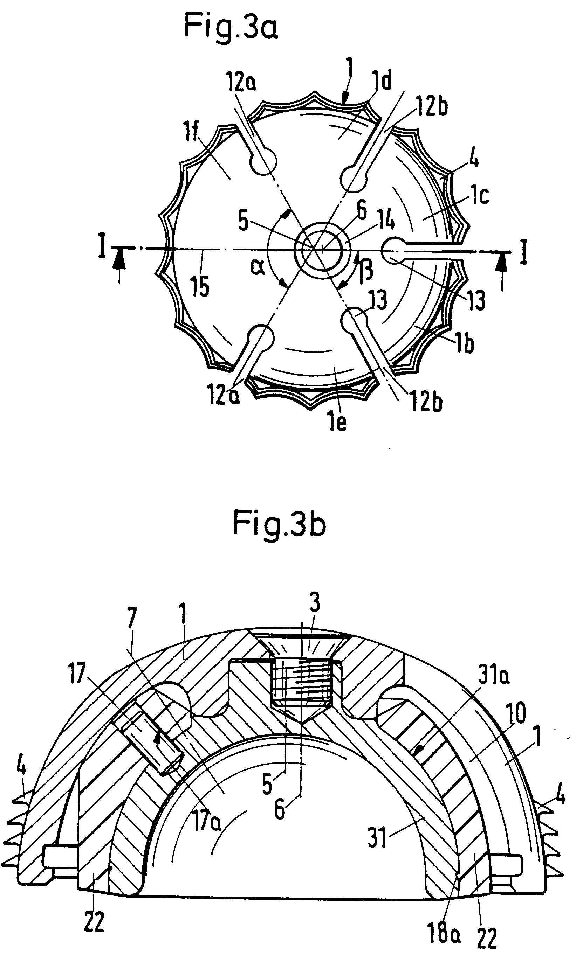

Die Figuren 3a und 3b zeigen ein weiteres Ausführungsbeispiel einer Hüftgelenkpfanne, die durch ein biologisch abbaubares Element 22 vorübergehend eine erhöhte Steifigkeit aufweist. Fig. 3a zeigt eine Aufsicht einer Aussenschale 1 in Richtung einer Montageachse 5. Die Aussenschale 1 weist über den Umfang verteilte Meridianschlitze 12a, 12b auf, die mit einer kreisförmigen Erweiterung 13 beginnen und bis zum Äquator der Aussenschale 1 auslaufen, sodass die Aussenschale 1 im Bereich des Äquators in Umfangsrichtung unterbrochen ist.
Die Meridianschlitze 12a, 12b unterteilen die Aussenschale 1 in Schalensegmente 1b-1f, wobei die Schalensegmente 1b-1e einen identischen Winkel β umfassen, wogegen das Schalensegment 1f einen Winkel alpha umfasst, der ungefähr doppelt so gross ist wie der Winkel β. Im Bereich der Hauptbelastungsrichtung 7 ist das eine vergrösserte Oberfläche aufweisende Schalensegment 1f angeordnet, da in diesem Bereich die grössten Kräft auf den Knochen zu übertragen sind. Die Innenschale 31 ist in Richtung der Montageachse 5 über ein Verbindungsmittel 3 mit der Aussenschale 1 verbunden, wobei die Achse 6 des Verbindungsmittels 3 zur Montageachse 5 versetzt liegt.
Die Elastizität der Aussenschale 1, beziehungsweise das Verhalten bei statischen und dynamischen Kräften, lässt sich durch die Dicke der Aussenschale 1 sowie durch die Anordnung der Meridianschlitze 12a, 12b über dem Umfang sowie deren Länge in weiten Grenzen variieren. Die Aussenschale ist üblicherweise symmetrisch aufgebaut mit einer Symmetrieebene entlang der Schnittfläche I-I.

Aus der Seitenansicht der Hüftgelenkpfanne entlang der Schnittfläche I-I ist in Fig. 3b ein Zwischenraum 10 zwischen Innenschale 31 und Aussenschale 1 ersichtlich, der teilweise mit einem biologisch abbaubaren, kugelschalenförmigen Element 22 gefüllt ist. Das Element 22 liegt auf der Aussenfläche 31a der Innenschale 31 auf, wobei das Element 22 im Bereich des Äquators einen Vorsprung 18b aufweist und die Innenschale 31 eine Kerbe 18a, sodass das Element 22 über einen derart gebildeten Schnappverschluss auf der Innenschale 31 befestigbar ist.
Weiter ist es möglich in der Innenschale 31 sowie im Element 22 eine Ausnehmung 17a für ein Verbindungsmittel 17 vorzusehen, um ein gegenseitiges Verdrehen von Innenschale 31 und Element 22 zu verhindern. Im Bereich der Hauptbelastungsrichtung 7 beziehungsweise im Bereich des Schalensegmentes 1f ist das Element 22 derart ausgestaltet, dass es sowohl auf der Innenschale 31 als auch auf der Aussenschale 1 aufliegt. Ein Teil der vom Kugelgelenkkopf auf die Innenschale 1 ausgeübten Kraft ist somit über das Element 22 auf die Aussenschale 1 übertragbar. Das statische und dynamische Verhalten der Kraftübertragung zwischen Innenschale 31 und Aussenschale 1 lässt sich durch die Elastizität sowie die Form des Elementes 22 beeinflussen. Im Bereich des Segmentes 1c füllt das Element 22 nicht den gesamten Zwischenraum 10 aus, wobei das Element 22 auf der Innenschale 31 aufliegt.
In diesem Bereich übt das Element 22 keinen Einfluss auf die Kraftübertragung zwischen der Innenschale 31 und der Segment 1c, ausser dass das Element 22 den maximalen Auslenkweg des Segmentes 1c begrenzen könnte. Ein Element 22 kann auch dazu dienen, den Zwischenraum 10 vollständig auszufüllen oder zumindest gegen aussen vollständig abzudichten, sodass der Zwischenraum 10 abgeschlossen ist.
Das Element 22 ist auch derart ausgestaltbar, dass es nebst dem Zwischenraum 10 auch die in meridialer Richtung verlaufenden Schlitze 12a, 12b teilweise oder vollständig ausfüllt. Das biologisch abbaubare Element 22 verleiht der Hüftgelenkpfanne während einer der Implantation folgenden Zeitperiode eine erhöhte Steifigkeit. Mit zunehmenden Abbau des Elementes 22 erlangt die Hüftgelenkpfanne ihre elastischen Eigenschaften wieder zurück.

Die dargestellten Ausführungsformen biologisch abbaubarer Elemente zur vorübergehenden Erhöhung der Steifigkeit einer orthopädischen Prothese sind nur als Beispiele zu verstehen aus einer Vielfalt möglicher Ausführungsformen.

## Patentansprüche

1. Orthopädische Prothese (1,10), welche mit einer Ausnehmung (2,11) versehen ist, dadurch gekennzeichnet, daß in der Prothese ein biologisch abbaubares Element (20,21,22) form- oder kraftschlüssig angeordnet ist zur vorübergehenden Erhöhung der Steifigkeit der Prothese (1,10), wobei das biologisch abbaubare Element (20,21,22) allmählich seine Lastaufnahmefähigkeit verliert, sodass die Prothese (1,10) einige Zeit nach der Implantation zunehmend elastischere Eigenschaften aufweist.

2. Orthopädische Prothese nach Anspruch 1, bei welcher das biologisch abbaubare Element (20,21,22) eine einfach zusammenhängende Form aufweist, die der Ausnehmung (2,11) der Prothese (1,10) angepasst ist.

3. Orthopädische Prothese nach einem der vorangehenden Ansprüche, bei welcher das biologisch abbaubare Element (22) sowie die Ausnehmung (11) der Prothese (1,10) gegenseitig angepasste Verbindungsmittel (11a, 22a) aufweisen, um das biologisch abbaubare Element (20,21,22) sowohl auf Zug als auch auf Druck zu belasten.

4. Orthopädische Prothese nach einem der vorangehenden Ansprüche, bei welcher das biologisch abbaubare Element (20,21,22) aus einem biologisch resorbierbaren Polymer besteht.

5. Orthopädische Prothese nach Anspruch 4, bei welcher das biologisch resorbierbare Polymer aus einer Gruppe ausgewählt ist, die aus Polyesteramid, Polydioxanon, Polymilchsäure, Polyglykolsäure, Polycaprolacton und deren Copolymeren besteht.

## Claims

1. An orthopaedic prosthesis (1,10) is provided with a recess (2,11), characterised in that a biodegradable element (20,21,22) is arranged positively connected or form-lockingly for temporarily increasing the rigidity of the prosthesis (1,10), and where the bio-degradable element (20,21,22) gradually loses its load bearing ability so that some time after implantation, the prosthesis (1,10) has increasing elastic properties.

2. An orthopaedic prosthesis according to claim 1, where the bio-degradable element (20,21,22) has a simple coherent shape which is adjusted to the prosthesis (1,10) recess (2,11).

3. An orthopaedic prosthesis according to any of the above claims, where the bio-degradable element (22) and likewise the prosthesis recess (11) have mutually adjusted connecting means (11a,22a) so as to load the bio-degradable element (20,21,22) with both push and pull forces.

4. An orthopaedic prosthesis according to any of the above claims, where the bio-degradable element (20,21,22) is comprised of a biologically reabsorbable polymer.

5. An orthopaedic prosthesis according to claim 4, where the biologically reabsorbable polymer is selected from a group comprising polyesteramide, polydioxanon, poly lactic acid, polyglycol acid, polycaprolacton and their co-polymers.

## Revendications

1. Prothèse orthopédique (1, 10) qui est pourvue d'un évidement (2, 11), caractérisée en ce qu'il est disposé par concordance des formes ou par force dans la prothèse un élément (20, 21, 22) biologiquement dégradable en vue d'une augmentation passagère de la rigidité de la prothèse (1, 10), où l'élément (20, 21, 22) biologiquement dégradable perd progressivement son aptitude à la réception d'une charge de telle sorte que la prothèse (1, 10), quelque temps après l'implantation, présente des caractéristiques de plus en plus élastiques.

2. Prothèse orthopédique selon la revendication 1, où l'élément (20, 21, 22) biologiquement dégradable a une forme cohérente simple qui est adaptée à l'évidement (2. 11) de la prothèse (1, 10).

3. Prothèse orthopédique selon l'une des revendications précédentes, où l'élément biologiquement dégradable (22) ainsi que l'évidement (11) de la prothèse (1, 10) présente des moyens de liaison (11a, 22a) adaptés mutuellement pour charger l'élément biologiquement dégradable ( 20, 21, 22) à la fois en traction et en pression.

4. Prothèse orthopédique selon l'une des revendications précédentes, où l'élément biologiquement dégradable (20, 21, 22) est constitué d'un polymère biologiquement résorbable.

5. Prothèse orthopédique selon la revendication 4, où le polymère biologiquement résorbable est choisi dans un groupe constitué de polyesteramide polydioxanone, acide polylactique, acide polyglycolique, polycaprolactone et leurs copolymères.
